# EUROPEAN PATENT APPLICATION

(11) **EP 2 201 980 A1**
(43) Date of publication of application: **30.06.2010**
(21) Application number: 09151636.9
(22) Date of filing: 29.01.2009
(51) Int. Cl.: A61N 1/36, A61N 1/05, A61N 1/37

(54) **Implantable tissue stimulator**

(30) Priority: 23.12.2008 SE 0802667
(71) Applicant: St. Jude Medical AB, 175 84 Järfälla (SE)
(72) Inventor: Eckerdal, Johan, 74192, KNIVSTA (SE); Nilsson, Susanne, 14133, HUDDINGE (SE)

(57) **Abstract**

The invention relates to an implantable tissue stimulator, connectable to at least one electrode lead including at least one stimulation electrode to be arranged in connection to tissue. The tissue stimulator comprises a stimulation pulse generator for generating pulses to be applied to the tissue via the at least one stimulation electrode and a controller. The stimulation pulse generator is further adapted to generate, in dependence of control signals from the controller, sub-threshold stimulation pulses to be applied to the tissue by the at least one stimulation electrode during a preset time period after implantation and fixation of the electrode(s).

## Description

### Field of the invention

The present invention relates to implantable tissue stimulators, such as spinal cord stimulators, deep brain stimulators, stomach stimulators, pacemakers, defibrillators, cardioverters, implantable cardioverter-defibrillators ("ICDs"), and similar tissue stimulation devices that may also be capable of monitoring and detecting electrical activities and events in tissue.

### Background of the invention

Electrical stimulation is used by a variety of different implantable medical devices. Some examples of these devices are implantable pacemakers and implantable cardioverters/defibrillators to control heart rhythm, spinal cord stimulators to block and control pain, deep brain stimulators for treating Parkinson's disease, and stomach stimulators to control transportation of food through the stomach. All these medical devices may be connected to chronically implanted electrodes. Stimulation pulses are emitted by the stimulator via the implanted electrodes to achieve the intended therapeutic effect. For example, an implantable heart stimulator generates electrical stimulation pulses and delivers such stimulation pulses using implantable leads to atrial and/or ventricular muscle tissue of a patient's heart at a prescribed rate and/or rhythm when the heart is not able to maintain the prescribed heart rate or rhythm on its own. When the delivered electrical stimuli are of sufficient energy, they cause the cardiac muscle tissue to depolarize, and therefore contract, thereby forcing the heart rate or rhythm to track the delivery of the electrical stimuli. When the delivered electrical stimuli are of insufficient energy, depolarization does not occur, and the heart rate or rhythm is not controlled by the pacemaker. Hence, for the implantable heart stimulator to perform its intended function, it is important that the delivered electrical stimuli are of sufficient energy to depolarize the cardiac tissue.

The implanted leads normally comprise one or more electrodes, arranged to measure the impedance or transmit pacing pulses generated by a pace pulse generator under influence of a controller. Any foreign material implanted in the body will be encapsulated by fibrous tissue, in this case the electrodes. The fibrous tissue layer will increase the distance from the electrode to viable tissue. This results in a lower current density in the tissue by the delivered stimulation pulses. As a consequence, the stimulation pulse energy threshold will increase to achieve the intended therapeutic effect. To compensate for the increased threshold, the stimulator pulse energy output needs to be increased. This will result in a shortened battery life time in the stimulator, or in worst case that the stimulation pulses do not provide the intended effect.

It is thus of high importance that the electrodes grow well into the tissue to minimize the thickness of the fibrotic tissue encapsulation on the electrode, to get a good conductivity to the tissue in order to achieve lowest possible energy threshold to provide the intended therapy . This threshold is in this context referred to as the stimulation threshold for therapeutic effect.

Some tissue stimulators include some kind of method to ensure that the given stimulation has the intended therapeutic effect. It is for example known to use results of impedance measurements to adjust stimulation parameters to achieve therapeutic effect by the stimulation. Impedance variations due to fibrotic tissue presence around electrode-nerve interface is discussed in "Implantable measurement technique dedicated to the monitoring of electrode-nerve contact in bladder stimulators", C. Donfack et al, Medical & Biological Engineering & Computing 2000, Vol. 38. Malfunctions of the electrode-nerve are here detected by impedance measurements with an "electrode-nerve contact verification circuit" and the variation in impedance can be used to adjust stimulation parameters to achieve the threshold of bladder enervation.

The depolarization and ensuing contraction of the muscle tissue in response to a delivered stimulation pulse is generally referred to in the art as "capture" and is thus the stimulation threshold for therapeutic effect in muscle tissue. At the time of lead implantation, the stimulation threshold for therapeutic effect is usually low. However, the presence of a foreign material normally induces an inflammatory response which increases the stimulation threshold for therapeutic effect substantially during the first few weeks after implantation. Poor healing of the tissue results in a thick scar tissue, i.e. a fibrous capsule formation, around the implanted lead. When the acute inflammatory response declines, the stimulation threshold for therapeutic effect usually return to a chronically stable low value, but still remain higher than it was at the time of implantation due to the fibrous capsule formation. The implantable tissue stimulator is then required to supply stimulation pulses of higher energy to be able to stimulate through the thick scar tissue.

Known techniques to minimize the formation of fibrous tissue encapsulation are to make the electrodes steroid eluting or to manufacture the electrodes in a highly biocompatible material (see e.g. US application 2008/0234790) that minimizes foreign body reaction. However, in relation to steroid eluting electrodes it is difficult to administer the right dosage and assure release during a prolonged period of time (up to 4 weeks is normally beneficial).

Furthermore, the international publication WO-2008/066423 describes treatment of cardiac tissue of a heart with therapeutic light using an implanted medical device, to improve the healing of the trauma following the implantation of the cardiac electrode into the cardiac tissue.

Electrical stimulation as a way to alleviate an inflammatory reaction is a known method in medicine for wound healing, especially in the treatment of chronic ulcers. The body has its own bioelectric system which influences wound healing by attracting the cells of repair, changing cell membrane permeability, enhancing cellular secretion through cell membranes and orienting cell structures. According to "Electrical stimulation for wound healing", chapter 16, Sussman et al., Aspen publishers 1998, electrical stimulation mimics the natural bioelectric system and jump start or accelerate the wound healing process. Electrical stimulation has further been shown to speed up wound healing by increasing capillary density and perfusion, improving wound oxygenation, and encouraging granulation and fibroblast activity, see Kloth, L, Nursing, December 2002. Further on, Zhao et al reported 2003 that electrical fields of small physiological magnitude (sub-threshold for muscle activity) directly stimulated VEGF production by endothelial cells, a key factor for angiogenesis, see Zhao et al, Journal of Cell Science 117 (3), 2003.

### Summary of the invention

The object of the present invention is to provide a technique to obtain a more viable tissue around stimulating electrodes. A further aim of the invention is to reduce the formation of fibrotic tissue around the stimulating electrodes.

The inventors have through experiments found that low energy stimulation below the threshold for therapeutic effect during the first several weeks after electrode implantation increases the formation of blood vessels in the tissue surrounding the lead. This stimulation regime is referred to as sub-threshold stimulation. The effect is achieved without the use of drug elution or the use of particularly biocompatible materials.

By improving the electrode ingrowth to the tissue, a low chronic stimulation threshold level for therapeutic effect is obtained which makes it possible to stimulate the tissue at a low energy level. This will e.g. result in a reduced power consumption of an implantable tissue stimulator.

The above-mentioned object is achieved by an implantable tissue stimulator, connectable to at least one electrode lead including at least one stimulation electrode to be arranged in connection to tissue. The tissue stimulator comprises a stimulation pulse generator for generating pulses to be applied to the tissue via the at least one stimulation electrode and a controller, wherein the stimulation pulse generator is adapted to generate, in dependence of control signals from the controller, sub-threshold stimulation pulses to be applied to the tissue by the at least one stimulation electrode during a preset time period after implantation and fixation of the electrode(s).

The present invention further improves the access to the human defence system via blood to the lead tip/muscle interface, by increasing the vascularization around the implanted electrode.

It is further an aim of the present invention to allow for a simplified design of the leads, and also for simplification of the regulatory pathways (as steroid usage is strongly regulated by the FDA - Food and Drug Association).

The present invention provides a new enhanced way to improve electrode ingrowth into tissue and maintaining low threshold throughout the acute phase and allow for a lower chronic threshold. The invention utilizes the capability of a pulse generator to emit electrical impulses through the lead tip. By applying low energy pulses, sub-threshold to stimulation threshold for therapeutic effect, the wound healing around the lead tip is enhanced and a more viable tissue is obtained.

Preferred embodiments are set forth in the dependent claims.

### Short description of the appended drawings

Figure 1 is a simplified illustration of an implantable stimulation device in electrical communication with at least one lead implanted into a patient's heart for delivering multichamber stimulation and shock therapy.
Figure 2 is a simplified illustration of an implantable stimulation device in electrical communication with at least one lead implanted into a patient's stomach for delivering gastric electrical stimulation.
Figure 3 is a simplified illustration of an implantable stimulation device in electrical communication with at least one lead implanted into a patient's spinal nervous tissue for delivering electrical stimulation to the spinal cord.
Figure 4 is a block diagram of the present invention.
Figure 5 is a schematic illustration of the increase in stimulation threshold for therapeutic effect during the acute-period.

### Detailed description of preferred embodiments of the invention

The present invention is applicable to any kind of implantable stimulation device which is in electrical communication with at least one lead implanted into tissue of a patient, but is in the following exemplified in conjunction with implantable stimulation devices intended mainly for heart stimulation, gastric stimulation and spinal cord stimulation.

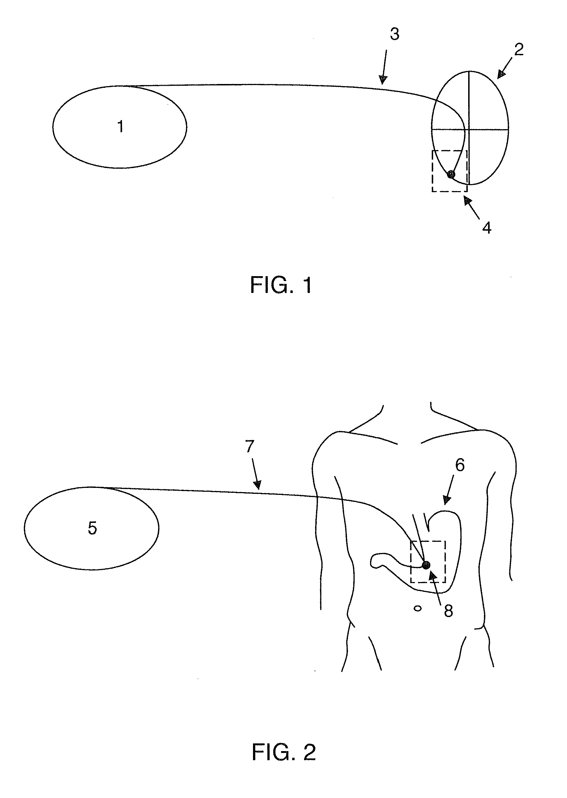

Fig. 1 illustrates an example of an implantable heart stimulator 1. The implantable heart stimulator 1 is in electrical communication with a patient's heart 2 by way of at least one lead 3. In order to sense cardiac signals and to provide stimulation therapy, the at least one lead is having a distal electrode lead tip electrode 4 arranged in connection to the heart tissue.

In fig. 2 an implantable gastric stimulator 5 is illustrated. The implantable gastric stimulator 5 is in electrical communication with a patient's stomach 6 by way of at least one lead 7 suitable for delivering gastric stimulation intended for e.g. assisting gastric emptying. The at least one lead 7 is provided with at least one electrode 8 arranged in connection to gastric tissue, but may also be arranged in connection to bowel.

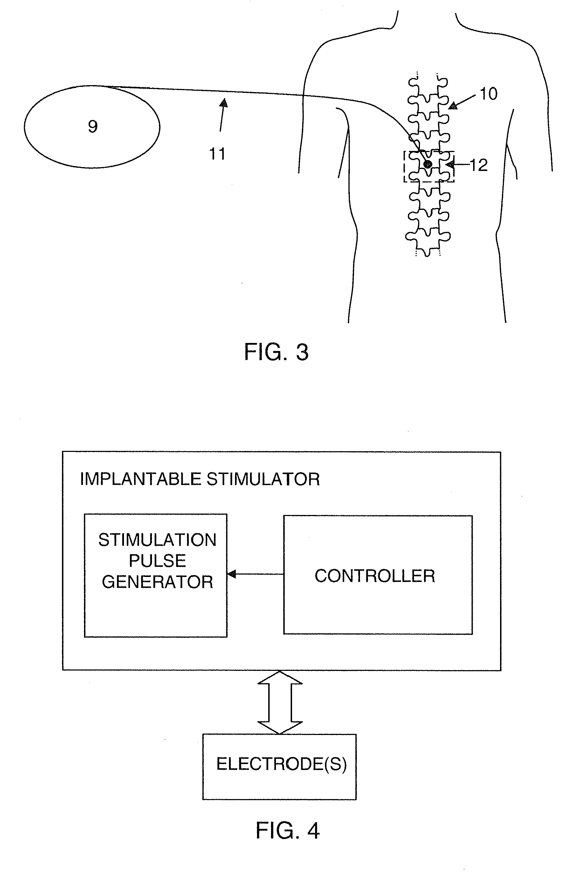

Fig. 3 illustrates an implantable spinal cord stimulator 9. The implantable spinal cord stimulator 9 is in electrical communication with a patient's spinal cord 10 by way of at least one lead 11 suitable for delivering spinal cord stimulation intended to block and control pain in different parts of the patient's body. The at least one lead 11 is provided with at least one electrode 12 arranged in connection to spinal cord tissue.

There is a plurality of different electrode leads that may be utilized in conjunction with the present invention. In a typical endocardial pacemaker, a first electrode lead having an electrode at its distal end is positioned to pace and sense the ventricle, and a second electrode lead having an electrode at its distal end is positioned to pace and sense the atrium. In a defibrillator system, a defibrillation electrode is used to deliver high voltage defibrillation countershocks to the ventricle and/or atrium. The invention may also be used in conjunction with epicardial electrodes. In addition to tip electrodes, ring electrodes or other kinds of electrodes may be utilised in conjunction with the present invention.

When stimulating the stomach, serosal electrodes or intraluminal ring electrodes may for example be used. Percutaneous and laminotomy leads or electrodes are examples of leads or electrodes used for spinal cord stimulation. A percutaneous lead may have three or more equally spaced electrodes. A laminotory lead typically have a paddle configuration and possesses a plurality of electrodes (for example two, four, eight or sixteen) arranged in one or more independent columns, i.e. a multi surface electrode. Electrical stimulation may be administered using any of the electrodes independently, and multiple leads may be used to address different distant spinal cord zones (see e.g. US patent No. 6,236,892).

The tissue stimulator 1 according to the present invention comprises a stimulation pulse generator for generating pulses to be applied to the tissue via at least one stimulation electrode and a controller controlling the transmitting of pulses from the stimulation pulse generator (see fig. 4).

According to one embodiment of the present invention, the stimulation pulse generator is adapted to generate, in dependence of control signals from the controller, sub-threshold stimulation pulses to be applied to the tissue by the at least one stimulation electrode during a preset time period after implantation and fixation of the electrode(s). The electrode(s) may be fixated be means of wedges, collars, tines, fins, a helifix, a basket, a screw, a needle or any other kind of fixation means, to the tissue. Thus, with the present invention it is possible to enhance the wound healing of the tissue around an electrode after implantation and to get a low and stable chronic threshold. The longevity of the device is also prolonged, as the implantable tissue stimulator may generate stimulation pulses at a lower energy level than before, as the chronic threshold is lower.

By enhancing the wound healing in the tissue around an implanted electrode, more viable tissue with more blood vessels, myocytes, neurocytes and/or other appropriate cell types of the specific tissue may be present around the electrode than before. This offers improved possibilities to create stable sensors on the electrode surface, as the signals intended to be received by the sensors, or signals that are emitted from the sensors, have to reach through less fibrous tissue than before. For example, a pO2-sensor would greatly benefit from being close to blood vessels, a pressure sensor would benefit from being encapsulated in softer tissue than more rigid fibrous tissue, a light-sensor would benefit from reflecting on intended tissue instead of on fibrous tissue etc. It is easy to depict several more examples where it is advantageous to have more viable tissue around the implanted electrode.

The present invention is naturally also applicable for multi-surface electrodes. The sub-threshold stimulation pulses may then be applied sequentially or in a parallel way to the tissue from the several surfaces of the electrode, e.g. either applying sub-threshold stimulation pulses separated in time or at the same time from different surfaces of the same electrode.

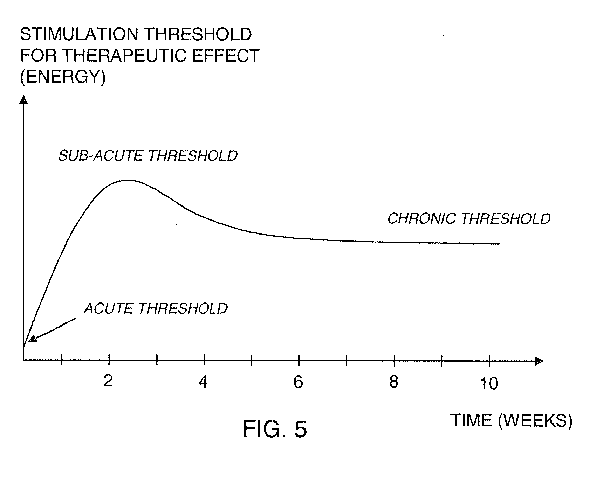

Fig. 5 schematically illustrates a typical change of the stimulation threshold for therapeutic effect during the acute period, i.e. the period following an implantation of a tissue stimulator. During the first approximately two weeks there is a rapid increase in the stimulation threshold for therapeutic effect (sub-acute threshold). Thereafter, the stimulation threshold for therapeutic effect decreases and reaches a plateau after approximately another four weeks (chronic threshold). The duration and size differs from patients to patient and is related to a variety of physiological and pharmacologic factors.

As the present invention may replace the use of steroid plugs (MCRD) or other steroid carriers at the lead tip, a simplified design of the leads may be achieved as well as simplified regulatory pathways. The present invention may thus be used in conjunction with steroid release tips, as explained further below.

According to one embodiment, the sub-threshold stimulation pulse has a pulse energy determined in relation to a stimulation threshold energy determined by a stimulation threshold algorithm. This algorithm may be a previously known and already implemented function to determine if the emitted stimulation pulses from the implanted stimulator has the intended therapeutic effect. Such known functions are an "Autocapture function" used in an implanted heart stimulator, or an "electrode-nerve contact verification circuit" used in an implantable bladder stimulator. For other types of devices other types of algorithms may be applicable. By measuring varying electrode-nerve contact impedance after implantation of the electrode(s), it is possible to determine the stimulation threshold for therapeutic effect and to adjust the sub-threshold stimulation accordingly. When detecting capture of heart tissue in relation to a cardiac chamber, a sensing circuitry may check for the depolarization of the heart tissue following and in response to a delivered stimulation pulse. Such a depolarization as a result of a delivered stimulation pulse is also referred to as an "evoked response" (ER) of that chamber. Furthermore, the evoked response is detected during a selected time period following the delivery of a stimulation pulse. Such a time period is generally referred to as an "evoked response window". Thus, it is possible to apply sub-threshold pulses that are proportional to the applied stimulation pulses to e.g. avoid applying sub-threshold stimulating pulses that may influence contraction of the heart or stomach, or may influence the pain alleviating stimulation to the spinal cord. The sub-threshold stimulation pulse energy may e.g. be 10-50 % of the stimulation threshold energy. Advantageously, the sub-threshold stimulation pulse energy is preferably lower than the determined stimulation threshold energy. The stimulation threshold algorithm may be used to monitor and advantageously assure that no stimulation above the threshold for therapeutic effect is achieved by the applied sub-threshold stimulation pulses i.e. if they in magnitude will be in proximity to the actual value of the stimulation threshold for therapeutic effect. There are of course other ways to determine a stimulation threshold energy for therapeutic effect, for example may other electrodes be used for sensing which stimulation energy that has to be used to obtain intended therapeutic effect.

The sub-threshold stimulation pulse energy is according to one embodiment varied during the time period in dependence of the stimulation threshold energy. Thus, if the stimulation threshold energy changes, the sub-threshold stimulation pulse energy is varied accordingly, to avoid applying too high pulse energy that would influence e.g. pacing or pain control, or to avoid applying pulses with unnecessary low energy. According to one embodiment when stimulating the heart, the sub-threshold stimulation pulses are applied only in the refractory periods of the heart. This will further increase the patient's safety.

The low energy pulses from an implantable heart stimulator have in experiments shown to have a profound effect on angiogenesis around the electrode after about three weeks of exposure. According to one embodiment of the invention, the sub-threshold stimulation pulse has a pulse amplitude of 0,05-0,6 V and a duration of 0,1-10 ms. These amplitude and duration intervals are preferably used in conjunction with a heart stimulator, but are also applicable when a gastric stimulator or spinal cord stimulator is employed.

Advantageously, the sub-threshold stimulation pulses are applied at a preset constant rate, e.g. 60 pulses per second. This rate may be the same as the stimulation frequency of the tissue stimulator i.e. one applied sub-threshold pulse at each stimulation interval (e.g. each heart cycle interval), or may come in recurrent couples or bursts during every stimulation interval (e.g. heart cycle interval). A predetermined number, e.g. 1-6, of sub-threshold stimulation pulses may thus be applied during each stimulation interval.

According to one embodiment, the sub-threshold stimulation pulse has a current in the range of 0,1-20 mA and a duration in the range of 0,2-600 ms. These intervals are advantageously used when applying gastric stimulation or spinal cord stimulation. The rate may, as explained above, be applied at a preset constant rate, for example the same as the stimulation frequency of the stimulator i.e. one applied sub-threshold pulse at each stimulation interval. At long intervals, the rate of the sub-threshold stimulation pulse is preferable greater than the stimulation frequency. A stimulation interval for gastric stimulation may be 0,1 s stimulation followed by 5 s without stimulation, and for spinal cord stimulation an example is 30 s of stimulation every 5 minute. These intervals are thus only examples, and many other intervals are possible. The sub-threshold stimulation pulse may also come in recurrent couples or bursts every stimulation interval. A predetermined number, e.g. 1-6, of sub-threshold stimulation pulses may thus be applied during each stimulation interval.

The rate of the sub-threshold stimulation pulses may thus according to one embodiment be different from the stimulation frequency to get desired effect. For example, the sub-threshold stimulation pulses may be applied during every second heart cycle interval, stimulation interval to the stomach or spinal cord, or may not be related to the stimulation interval at all. The possible rate is according to one embodiment from one pulse every fifth second (12 pulses per minute) up to one pulse every 1/100 second (6000 pulses per minute). The implantable tissue stimulator may thus emit sub-threshold electric pulses uni- or bipolar to the lead tip electrodes at a predetermined amplitude and frequency. The waveform of the pulse is according to one embodiment also predetermined.

According to one example, 0,3 V was applied with 1 ms interval at 60 bpm, and the pulse was confirmed to not give any muscle stimulation. A plurality of electrodes were implanted, some applying low energy pulses and some not applying any pulses at all, being control electrodes. After 3 weeks, the electrodes were removed and the area around the implant site was carefully examined. It was found that the fibrous capsule around the electrodes that had been applying low energy pulses was more vascularized than around the control electrodes, with the average 14 vessels found around an electrode that had been applying low energy pulses, versus 2 vessels around a control electrode. In total 9.1 % of the area of the fibrous capsule were vessels in comparison to 1.9% of the area of the control electrode. In addition to improved wound healing (with reduced fibrous capsule formation as a consequence), a low and stable stimulation threshold for therapeutic effect is achieved.

A further effect of the invention is that the capsule around an electrode applying low-energy pulses gets a better oxygen transport, CO₂-transport and nutrition. The immune system is dependent of good vascularisation as leukocytes and humoral defense mechanisms come to a site of infection via blood. It is therefore likely that if any bacterial cells come with the lead tip, there will be a better chance for the immune system to take care of them if the present invention has been utilized.

According to one embodiment, the sub-threshold stimulation pulses are generated at a variable rate. This rate may be determined in dependence of the rate of the stimulation pulses, or may be varied in accordance with the elapsed time during the time horizon. For example, two weeks after implementation and applying of sub-threshold stimulation pulses with a certain rate, the rate may be declining for a certain time and finally stopped to give the best effect possible.

According to one embodiment, the preset time period is variable. Advantageously, the preset time period is set to 2-8 weeks, and more preferably 2-4 weeks. It has been found that application of sub-threshold stimulation pulses is most beneficial during these weeks after implementation of the electrodes in the tissue.

The present invention includes, according to one embodiment, a "Threshold enhancer/wound healing" algorithm that is activated by a user, a nurse or a physician, at the time of implant. The algorithm may be implemented as executable program code in the controller, and when executed performs the necessary steps for applying sub-threshold stimulation pulses to the tissue according to the invention. The controller thus comprises some kind of processing means. The algorithm may function totally independent from conventional stimulating capabilities of the implantable tissue stimulator.

The implantable tissue stimulator may according to one embodiment be used in conjunction with at least one electrode lead provided with a steroid releasing means arranged in connection with the electrode(s). Hence, the effect of the released steroids and the applied sub-threshold stimulation pulses may together enhance the healing of the tissue.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Implantable tissue stimulator (1, 5, 9), connectable to at least one electrode lead (3, 7, 11) including at least one stimulation electrode (4, 8, 12) to be arranged in connection to tissue, said tissue stimulator comprises
a stimulation pulse generator for generating pulses to be applied to the tissue via said at least one stimulation electrode,
a controller,
**characterized in that** said stimulation pulse generator is adapted to generate, in dependence of control signals from said controller, sub-threshold stimulation pulses to be applied to the tissue by said at least one stimulation electrode during a preset time period after implantation and fixation of said electrode(s).

2. Implantable tissue stimulator according to claim 1, wherein said sub-threshold stimulation pulse has a pulse energy determined in relation to a stimulation threshold energy determined by a stimulation threshold algorithm.

3. Implantable tissue stimulator according to claim 2, wherein said sub-threshold stimulation pulse energy is lower than said determined stimulation threshold energy.

4. Implantable tissue stimulator according to claim 2, wherein said sub-threshold stimulation pulse energy is varied during said time period in dependence of the stimulation threshold energy.

5. Implantable tissue stimulator according to anyone of the preceding claims, wherein said sub-threshold stimulation pulse has a pulse amplitude of 0,05-0,6 V and a duration of 0,1-10 ms.

6. Implantable tissue stimulator according to any of claims 1 to 4, wherein said sub-threshold stimulation pulse has a current of 0,1-20 mA and a duration of 0,2-600 ms.

7. Implantable tissue stimulator according to anyone of the preceding claims, wherein said sub-threshold stimulation pulses are applied at a preset constant rate, e.g. 60 pulses per second.

8. Implantable tissue stimulator according to anyone of the preceding claims, wherein said sub-threshold stimulation pulses are applied at a variable rate.

9. Implantable tissue stimulator according to anyone of the preceding claims, wherein said preset time period is variable.

10. Implantable tissue stimulator according to anyone of the preceding claims, wherein said preset time period is set to 2-8 weeks.

11. Implantable tissue stimulator according to anyone of the preceding claims, wherein said preset time period is set to 2-4 weeks.

12. Implantable tissue stimulator according to anyone of the preceding claims, wherein said sub-threshold stimulation pulses are applied only in the refractory periods of a heart.

13. Implantable tissue stimulator according to anyone of the preceding claims, wherein the stimulator is any of an implantable pacemaker, an implantable cardioverter/defibrillator, a spinal cord stimulator, deep brain stimulator or a stomach stimulator.
